# EUROPÄISCHE PATENTANMELDUNG

(11) **EP 2 529 683 A1**
(43) Veröffentlichungstag der Anmeldung: **05.12.2012**
(21) Anmeldenummer: 11168177.1
(22) Anmeldetag: 31.05.2011
(51) Int. Cl.: A61B 17/34

(54) **Trokarhülse**

(71) Anmelder: Karl Storz GmbH & Co. KG, 78532 Tuttlingen (DE)
(72) Erfinder: Oberländer, Martin, 78532 Tuttlingen (DE); Tan, H.L., Parkville Melbourne, VIC 3052Ro (AU)

(57) **Zusammenfassung**

Die Erfindung betrifft eine Trokarhülse für die minimal-invasive Chirurgie, mit einem ersten Hülsenteil (4), der im Wesentlichen die Form eines geraden Rohrstücks mit einer Längsachse hat, und mit einem zweiten Hülsenteil (6), der den ersten Hülsenteil (4) eng anliegend mindestens teilweise umgibt und im Gebrauch relativ zu dem ersten Hülsenteil (4) beweglich ist. Gemäß der Erfindung besteht die Beweglichkeit des zweiten Hülsenteils (6) relativ zu dem ersten Hülsenteil (4) im Wesentlichen nur in einer Drehbarkeit des zweiten Hülsenteils (6) um den ersten Hülsenteil (4) herum, und außerdem weisen der erste Hülsenteil (4) und der zweite Hülsenteil an ihnen distalen axialen Enden jeweils einen Flanschteil (14, 16) auf, der sich über einen Winkel von weniger als 180° von dem jeweiligen Hülsenteil (4, 6) radial nach außen erstreckt.

## Beschreibung

Die Erfindung betrifft eine Trokarhülse für die minimal-invasive Chirurgie, mit einem ersten Hülsenteil, der im Wesentlichen die Form eines geraden Rohrstücks mit einer Längsachse hat, und mit einem zweiten Hülsenteil, der den ersten Hülsenteil eng anliegend mindestens teilweise umgibt und im Gebrauch relativ zu dem ersten Hülsenteil beweglich ist.

Eine derartige Trokarhülse ist aus der WO 2010/136805 A1 bekannt, wobei die beiden Hülsenteile zwei teleskopartig ineinander und auseinander schiebbare gerade Rohrstücke sind, deren Relativverschiebung entweder geradlinig oder entlang einer Schraubenlinie erfolgt. Der radial äußere Hülsenteil ist an seinem proximalen Ende mit einem Kopfteil verbunden, der eine Dichtung zum gasdichten Einführen eines Instruments in die Trokarhülse und einen Fluidanschlussstutzen aufweist, und der radial innere Hülsenteil trägt an seinem distalen Ende einen flexiblen ringförmigen Flansch.

Eine Trokarhülse ist ein medizinisches Instrument, das in der minimal-invasiven Chirurgie zum Einführen von Instrumenten in den menschlichen oder tierischen Körper verwendet wird. Bei einem minimal-invasiven chirurgischen Eingriff wird ein Trokar, der aus einer Trokarhülse und einem in der Trokarhülse aufgenommenen Trokardorn besteht, zunächst dazu verwendet, einen Zugang zu einer Körperhöhle zu schaffen. Dazu wird die Spitze des Trokardorns an einem Einschnitt an der Körperhaut angesetzt und dann durch die Körperdecke hindurchgestoßen. Anschließend wird der Trokardorn aus der Trokarhülse herausgezogen; die Trokarhülse bleibt im Körper stecken. Durch die Trokarhülse können dann abwechselnd Instrumente wie Endoskope, Zangen, Scheren, Nahtinstrumente und dergleichen zum Durchführen chirurgischer Maßnahmen in die Körperhöhle eingeführt werden.

Der flexible Flansch am distalen Ende einer Trokarhülse entfaltet sich unterhalb der durchstoßenen Körperdecke und kann dann von außen mit einem entsprechenden weiteren Flansch gekontert werden, um für eine sichere Verankerung an der Körperdecke zu sorgen. Damit sich der Flansch unter der Körperdecke entfalten kann, muss die Trokarhülse relativ tief in den Körper hinein geschoben werden, und außerdem muss ein flexibler Flansch aus Gummi oder dergleichen relativ dick sein, um ihn wirksam von außen kontern zu können, so dass der Flansch auch während eines Eingriffs relativ viel Raum unter der Körperdecke einnimmt. Außerdem sind die meisten Trokarhülsen für eine gewisse Mindestdicke der Körperdecke ausgelegt. Aus diesen Gründen sind die bekannten Trokarhülsen für einige Operationen nicht geeignet, z. B. Operationen an Kleinkindern oder an der Schilddrüse.

Der Erfindung liegt die Aufgabe zu Grunde, eine Trokarhülse bereitzustellen, die eine geringe Einführtiefe und keine Mindestdicke der Körperdecke benötigt und die auch einfach herzustellen, leicht zu montieren und gut zu reinigen ist.

Diese Aufgabe wird durch eine Trokarhülse mit den Merkmalen des Anspruchs 1 gelöst. Vorteilhafte Weiterbildungen der Erfindung sind in den abhängigen Ansprüchen angegeben.

Gemäß der Erfindung weisen die beiden Hülsenteile an ihren distalen axialen Enden jeweils einen Flanschteil auf, der sich über einen Winkel von weniger als 180° von dem jeweiligen Hülsenteil radial nach außen erstreckt. Wenn die Hülsenteile in eine Relativposition gedreht werden, in der die beiden Flanschteile in dieselbe Richtung weisen und im Wesentlichen deckungsgleich übereinander liegen, können die beiden Flanschteile gemeinsam durch und unter die Körperdecke geschoben werden, ohne dass sie flexibel sein müssen, weil die Elastizität der Körperdecke selbst ausreicht. Dies gilt besonders dann, wenn die Flanschteile, wie bevorzugt, ungefähr U-förmige Umrisse haben, wie in einer Ebene senkrecht zu der Längsachse gesehen, wobei der Abstand der beiden Schenkel der U-Form ungefähr gleich dem Durchmesser des ersten oder vorzugsweise des zweiten Hülsenteils ist. Bevorzugt zeigen beide Flanschteile denselben Winkel von dem jeweiligen Hülsenteil.

Nach dem Einführen können die Hülsenteile von Hand leicht in eine Relativposition gedreht werden, in der die beiden Flanschteile, insesbesondere in einander entgegengesetzte Richtungen weisen und in einer Ebene senkrecht zu der Längsachse liegen, so dass die Trokarhülse an der durchstoßenen Körperdecke verankert ist.

Da die Flanschteile nicht flexibel sein müssen, können sie aus einem ebenso stabilen Material wie die Hülsenteile bestehen und sind vorzugsweise damit einstückig. Derartige Flanschteile können wesentlich dünner sein als ein flexibler Flansch, beispielsweise ungefähr 1 mm dick. In der Einführposition, in der die beiden Flanschteile direkt übereinander liegen, beträgt deren Gesamtdicke dann 2 mm, was immer noch vergleichsweise wenig ist, so dass die Trokarhülse eine sehr geringe Einführtiefe in einen Körper hat.

Gemäß der Erfindung besteht die Beweglichkeit des zweiten Hülsenteils relativ zu dem ersten Hülsenteil im Wesentlichen nur in einer Drehbarkeit des zweiten Hülsenteils um den ersten Hülsenteil herum. Im Hinblick auf das Vorangehende ist dies so zu verstehen, dass eine axiale Beweglichkeit, die nur dazu dient, den einen der beiden Flanschteile, die in der Einführposition übereinander liegen, im Verlauf der Drehung in die Arbeitsposition im Wesentlichen in dieselbe Ebene wie jene des anderen Flanschteils zu bringen, um eine plane Verankerungsfläche unter der Körperdecke zu erzeugen, unschädlich ist. In dem genannten Beispiel von 1 mm dicken Flanschteilen würde eine derartige Verschiebung 1 mm betragen. Ebenfalls unschädlich ist es, wenn es im Verlauf einer Drehung zwischen der Einführposition und der Arbeitsposition eine besondere Montageposition gibt, in der der zweite Hülsenteil auch axial verschoben werden kann, um ihn leicht von dem ersten Hülsenteil und dem Kopfteil lösen zu können. Es ist zweckmäßig, eine derartige Montageposition vorzusehen, um die Trokarhülse leicht zusammenzubauen und für eine Reinigung schnell wieder auseinander nehmen zu können.

Prinzipiell kann jeder der beiden Hülsenteilen im Wesentlichen, d. h. als einen Grundkörper, ein gerades Voll-Rohr aufweisen. In diesem Fall muss der erste, innere Hülsenteil lösbar am Kopfteil befestigt sein, beispielsweise mittels Überwurfmutter und Positionsstiften, damit der zweite Hülsenteil montiert und demontiert werden kann.

In einer alternativen Ausführungsform hat der am ersten Hülsenteil anliegende Abschnitt des zweiten Hülsenteils im Wesentlichen die Form einer Hälfte eines der Länge nach zweigeteilten Rohres. In diesem Fall kann der zweite Hülsenteil einfach axial über den ersten Hülsenteil und entlang der Längsachse in Richtung auf den Kopfteil der Trokarhülse geschoben werden und dann in die im Kopfteil ausgebildeten Führungs- und Verriegelungsmittel eingreifen gelassen werden.

Der Querschnitt des zweiten Hülsenteils senkrecht zu der Längsachse kann z. B. einfach ein Halbkreis sein.

Besser ist es, wenn der Querschnitt des zweiten Hülsenteils senkrecht zu der Längsachse ein Kreisbogen ist, der einige Winkelgrade mehr als ein Halbkreis hat. In diesem Fall umgibt der zweite Hülsenteil den ersten Hülsenteil über einen Winkel von etwas mehr als 180° und wird dadurch entlang seiner ganzen Länge formschlüssig am ersten Hülsenteil festgehalten, was auch für einen guten Zusammenhalt der beiden Hülsenteile im Gebrauch sorgt. Um das axiale Aufschieben des zweiten Hülsenteils auf den ersten Hülsenteil zu erleichtern, kann der Flanschteil des ersten Hülsenteils dort, wo er in den ersten Hülsenteil übergeht, mit kleinen Einkerbungen versehen sein.

Zum Kontern der Flanschteile auf der Außenseite der Körperdecke kann man grundsätzlich irgendein scheibenförmiges Element verwenden, insbesondere ein zweiteiliges Element, das nach der Montage des zweiten Hülsenteils rings um die Hülsenteile montiert werden kann.

Für die Erfindung besonders zweckmäßig ist jedoch ein Konterelement in Form einer einstückigen geschlitzten Gummischeibe, die bei geweitetem Schlitz von der Seite über die Hülsenteile der Trokarhülse geschoben werden kann und sich dann aufgrund ihrer Eigenelastizität mehr oder weniger fest um die Hülsenteile schließt. Nachdem man die Trokarhülse in der Körperdecke verankert hat, schiebt man die Gummischeibe einfach entlang der Längsachse in Richtung auf die Körperdecke, um die Flanschteile von außerhalb der Körperdecke zu kontern. Die Gummischeibe bleibt dann einfach durch Haftreibung in dieser Position.

Es folgt eine Beschreibung von Ausführungsbeispielen anhand der Figuren. Die Erfindung ist nicht auf die dargestellten Beispiele beschränkt. Es zeigen:
- Figur 1: eine Perspektivansicht einer Trokarhülse, deren äußerer Hülsenteil davon getrennt und in einer Position vor dem Zusammenbau ist;
- Figur 2: eine Perspektivansicht der Trokarhülse von Figur 1 in einer Montageposition, d. h. in einer Phase kurz bevor sie ganz zusammengebaut ist;
- Figur 3: eine Perspektivansicht der zusammengebauten Trokarhülse mit dem äußeren Hülsenteil in einer Einführposition, in der die Trokarhülse in eine in einer Körperdecke hergestellte Öffnung eingeführt werden kann;
- Figur 4: eine Perspektivansicht der zusammengebauten Trokarhülse mit dem äußeren Hülsenteil in einer Arbeitsposition, in der die Trokarhülse hinter einer in einer Körperdecke hergestellten Öffnung verankert sein kann;
- Figur 5: eine Perspektivansicht der Trokarhülse von Figur 4 mit einer aufgesteckten Gummischeibe als Konterelement; und
- Figur 6: eine Seitenansicht der Trokarhülse von Figur 5, wie sie in einer Körperdecke verankert und gekontert ist.

Die in den Figuren gezeigte Trokarhülse besteht aus einem Kopfteil 2, einem ersten, inneren Hülsenteil 4 und einem zweiten, äußeren Hülsenteil 6.

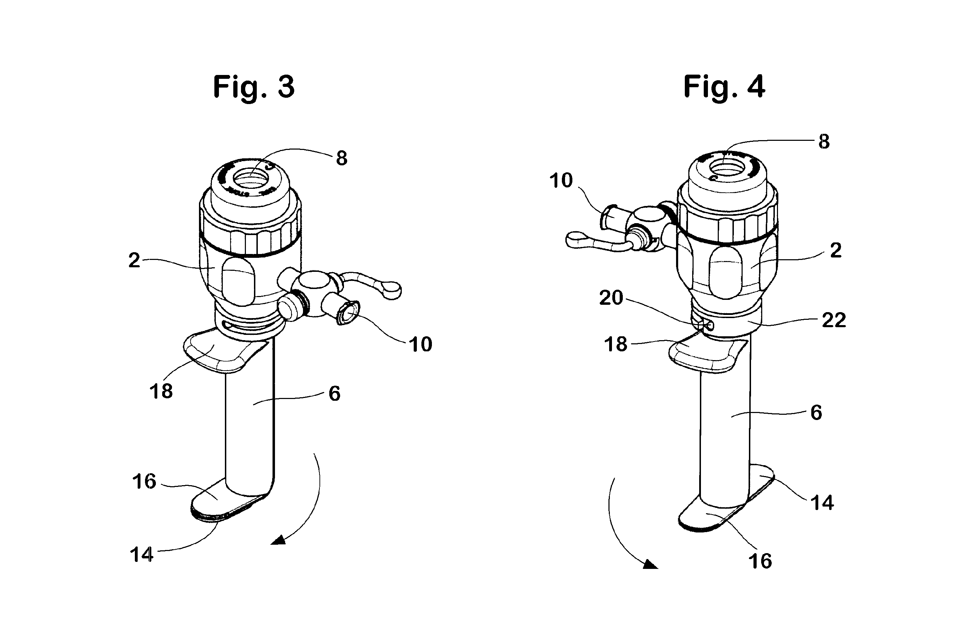

Der Kopfteil 2 ist ein ungefähr rotationssymmetrisches Gehäuse, das ein axiales Durchgangsloch enthält. In einem proximalen, in Figuren 3 bis 6 oberen Ende des Kopfteils 2 befindet sich eine flexible Dichtung 8 zum gasdichten Einführen eines Instruments in und durch das Durchgangsloch im Kopfteil 2. Außerdem hat der Kopfteil 2 einen Fluidanschlussstutzen 10 mit einem Ventil 12. Über den Fluidanschlussstutzen 10 können z. B. Insufflationsgas oder Spülflüssigkeit zugeführt werden.

Am distalen Ende des Kopfteils 2 ist ein proximales Ende des inneren Hülsenteils 4 befestigt, welcher hauptsächlich aus einem Stück Rohr besteht, dass sich in der axialen Verlängerung des Durchgangslochs im Kopfteil 2 bis zu einem distalen Ende erstreckt.

Ganz am distalen Ende des inneren Hülsenteils 4 ist ein erster Flanschteil 14 angeformt, der ungefähr die Form eines Spatels hat. Insbesondere hat der Flanschteil 14 einen U-förmigen Umriss, wie in einer Ebene senkrecht zur Längsachse des inneren Hülsenteils 4 gesehen, wobei der Abstand der beiden Schenkel der U-Form gleich dem oder etwas kleiner als der Durchmesser des inneren Hülsenteils 4 ist. Im Ausführungsbeispiel besteht der erste Flanschteil 14 aus demselben Material wie der innere Hülsenteil 4 und ist beispielsweise 1 mm dick.

Der äußere Hülsenteil 6 besteht hauptsächlich aus einem der Länge nach halbierten Stück Rohr etwa von derselben Länge und aus demselben Material wie der innere Hülsenteil 4. Der Querschnitt des äußeren Hülsenteils 6 senkrecht zu der Längsachse ist ein Kreisbogen, der einige Winkelgrade mehr als ein Halbkreis umfasst. Der Innendurchmesser des äußeren Hülsenteils 6 ist gleich dem Außendurchmesser des inneren Hülsenteils 4.

Am distalem Ende des äußeren Hülsenteils 6 ist ein zweiter Flanschteil 16 angeformt, der praktisch denselben Umriss und dieselbe Dicke wie der erste Flanschteil 14 hat und auf dieselbe Weise, wie der erste Flanschteil 14 einen rechtwinklig abknickenden Fortsatz des inneren Hülsenteils 4 bildet, einen radial nach außen verlaufenden und somit rechtwinklig abknickenden Fortsatz des äußeren Hülsenteils 6 bildet.

In der Nähe des proximalen Endes des äußeren Hülsenteils 6 ist ein Griffteil 18 daran angeformt, der ähnlich wie der zweite Flanschteil 16 einen radial nach außen verlaufenden Fortsatz des äußeren Hülsenteils 6 bildet, aber dicker und breiter ist. Noch etwas näher am proximalen Endes des äußeren Hülsenteils 6 ist ein kleiner runder Zapfen 20 daran angeformt, der sich vom äußeren Hülsenteil 6 einige Millimeter radial nach erstreckt.

Der Kopfteil 2 besitzt an seinem distalen Ende einen geschlitzten Ring 22, der sich in einem Abstand, der etwas größer als die Dicke des äußeren Hülsenteils 6 ist, radial um den inneren Hülsenteil 4 herum erstreckt. Im Zusammenwirken mit dem Zapfen 20 am äußeren Hülsenteil 6 bildet der geschlitzte Ring 22 Führungs- und Verriegelungsmittel für den äußeren Hülsenteil 6, und zwar nach Art eines Bajonettverschlusses, wie nachfolgend näher erläutert wird.

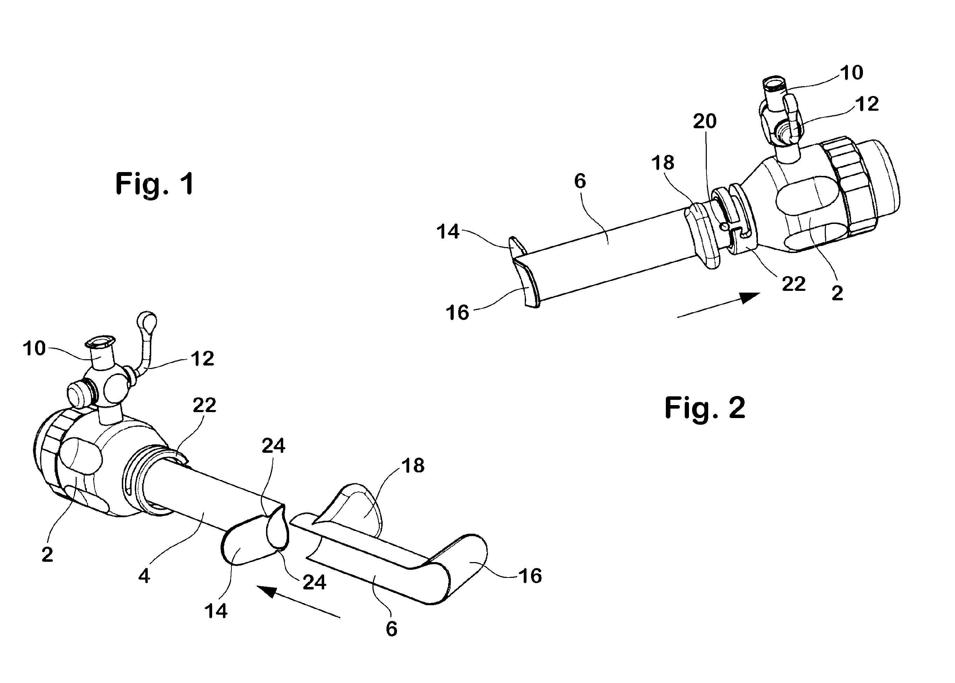

Wie in Figuren 1 und 2 veranschaulicht, wird die Trokarhülse montiert, indem der äußere Hülsenteil 6 in der eingezeichneten Pfeilrichtung auf den inneren Hülsenteil 4 aufgeschoben wird, wobei die beiden Flanschteile 14, 16 ungefähr in einander entgegengesetzte Richtungen weisen.

Um das Aufschieben zu erleichtern, hat der erste Flanschteil 14 zwei kleine Einkerbungen 24 in seinen Rändern, nämlich dort, wo er mit dem ersten Hülsenteil 4 verbunden ist. Die Einkerbungen 24 ermöglichen es auch, den äußeren Hülsenteil 6 um einige Winkelgrade zu verdrehen, wenn er auf dem inneren Hülsenteil 4 sitzt. Insbesondere kann man den äußeren Hülsenteil 6 in die in Figur 2 gezeigte Position drehen, in der die beiden Flanschteile 14, 16 in um ungefähr 160° versetzte Richtungen weisen, und dann den äußeren Hülsenteil 6 vollständig auf den inneren Hülsenteil 4 aufschieben.

Wenn der äußere Hülsenteil 6 vollständig auf den inneren Hülsenteil 4 aufgeschoben ist, wird der äußere Hülsenteil 6 um den inneren Hülsenteil 4 herum in die in Figur 3 gezeigte Position gedreht, in der die beiden Flanschteile 14 und 16 deckungsgleich übereinander liegen. Während der Drehung, deren Drehrichtung in Figur 3 mit einem Pfeil angezeigt ist, gleitet der Zapfen 20 in dem geschlitzten Ring 22 und wird dadurch geführt.

In der in Figur 3 gezeigten Position können die beiden Flanschteile 14 und 16 der Trokarhülse leicht in eine in einer Körperdecke hergestellte Öffnung eingeführt werden, ohne tief darin einzudringen, indem die Trokarhülse mehr oder weniger senkrecht über der Körperdecke gehalten und etwas nach unten und zur Seite bewegt wird, damit die beiden Flanschteile 14 und 16 gemeinsam unter die Körperdecke gleiten.

Nachdem die Trokarhülse ihre Endposition in und über der Körperöffnung erreicht hat, wird der äußere Hülsenteil 6 mit Hilfe des Griffteils 18 um den inneren Hülsenteil 4 herum zurückgedreht, nämlich in die in Figur 4 gezeigte Position, in der die beiden Flanschteile 14 und 16 in einander genau entgegengesetzte Richtungen weisen und genau in einer Ebene senkrecht zu der Längsachse der Hülsenteile 4 und 6 liegen. Die Drehrichtung ist in Figur 4 mit einem Pfeil angezeigt. In dieser Position wird der Zapfen 20 am ersten Hülsenteil 4 durch eine Hinterschneidung im geschlitzten Ring 22 festgehalten, so dass der zweite Hülsenteil 6 an der Trokarhülse verriegelt ist. In den durch seine Eigenelastizität gegebenen Grenzen ist der zweite Hülsenteil 6 auch entlang seiner Länge verriegelt, weil der äußere Hülsenteil 6 den inneren Hülsenteil 4 um mehr als 180° umgreift.

Die Führungswirkung des geschlitzten Rings 22 am Kopfteil ist entweder rein rotatorisch, oder sie bewirkt auch eine geringfügige Längsverschiebung von der Dicke der Flanschteile 14 und 16, wodurch der zweite Flanschteil 16 automatisch aus der in Figur 3 gezeigten Position, in der er deckungsgleich über dem ersten Flanschteil 14 liegt, in die in Figur 4 gezeigte Position versetzt wird, in der er genau in einer Ebene mit dem ersten Flanschteil 14 liegt. Ein derartiger Versatz kann aber auch durch entsprechende Ausbildung des Abschnitts des geschlitzten Rings 22 erzielt werden, in dem der Zapfen 20 durch eine Hinterschneidung im geschlitzten Ring 22 verriegelt wird.

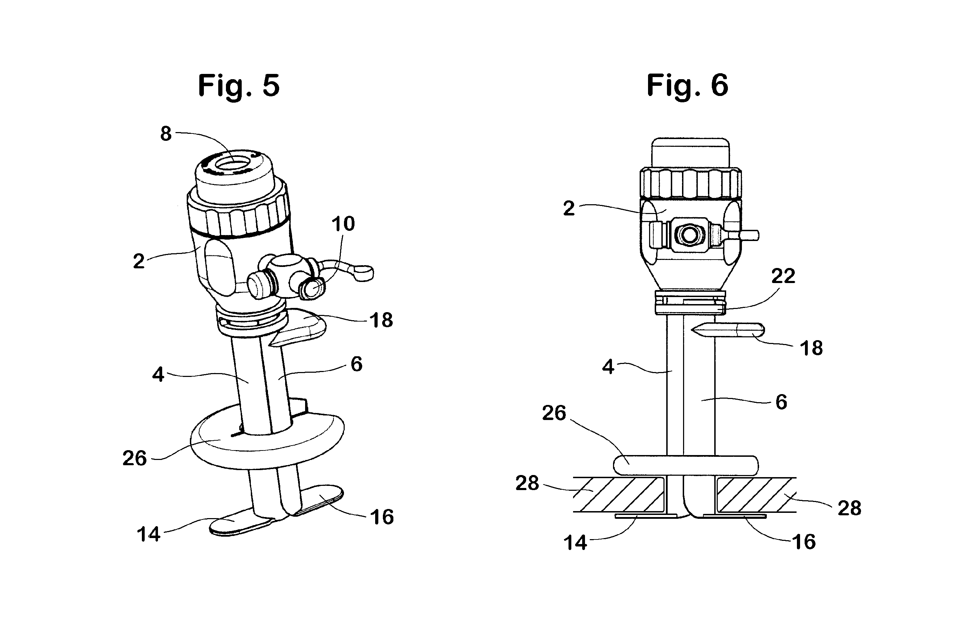

Wie in Figur 5 gezeigt, kann nach dem Verankern der Trokarhülse in einer Körperöffnung eine geschlitzte und von Hand auseinander ziehbare Gummischeibe 26 von der Seite über beide Hülsenteile 4 und 6 der Trokarhülse geschoben werden. Wenn sich die Gummischeibe 26 wieder zusammenzieht, umschließt sie die Hülsenteile 4 und 6 mit einer gewissen Kraft. Die Gummischeibe 26 kann dann von Hand in Richtung auf die Flanschteile 14 und 16 geschoben werden, bis eine in Figur 6 schematisch im Schnitt eingezeichnete Körperdecke 28 unten an den Flanschteilen 14, 16 und oben an der Gummischeibe 26 anliegt, so dass die Trokarhülse sicher an der Körperdecke 28 verankert ist.

Statt der Gummischeibe 26 kann man auch irgendein anderes geeignetes Konterelement verwenden, z. B. ein zweiteiliges Konterelement, das um die Hülsenteile 4 und 6 herum montiert werden kann.

In dem gezeigten Ausführungsbeispiel sind die Flanschteile 14 und 16 spatelförmig bzw. U-förmig. Obwohl diese spezielle Ausführungsform besonders vorteilhaft ist, was ein gewebeschonendes und nicht zu tiefes Einführen in die Körperdecke angeht, sind auch andere Umrissgestalten möglich. Beispielsweise können die Flanschteile 14 und 16 irgendeine Blattform haben, wie sie bei Pflanzenblättern vorkommt, doch sollten ihre Umrisse gerundet sein. Wesentlich ist, dass sich jeder Flanschteil 14, 16 über einen Winkel von weniger als 180° von dem jeweiligen Hülsenteil 4 bzw. 6 radial nach außen erstreckt.

In dem gezeigten Ausführungsbeispiel sind die Flanschteile 14 und 16 außerdem eben und liegen stets parallel zueinander. Sie könnten z. B. aber auch Kegelabschnitte oder Kugelabschnitte ausbilden, was für Operationen an eher konvexen oder konkaven Körperteilen vorteilhaft sein kann.

Ein weiteres, ebenfalls nicht gezeigtes Ausführungsbeispiel unterscheidet sich von dem Ausführungsbeispiel der Figuren 1 bis 6 im Wesentlichen darin, dass der Grundkörper des äußeren Hülsenteils keinen halbkreisförmigen Querschnitt hat, wie bei dem äußeren Hülsenteil 6, sondern ebenso wie bei dem inneren Hülsenteil 4 ein Voll-Rohr ist. Um in diesem Fall den äußeren Hülsenteil montieren und demontieren zu können, muss das distale Ende des inneren Hülsenteils lösbar am Kopfteil befestigt sein, z. B. mittels Überwurfmutter und Positionsstiften.

### Bezugszeichen

- 2: Kopfteil
- 4: erster, innerer Hülsenteil
- 6: zweiter, äußerer Hülsenteil
- 8: flexible Dichtung
- 10: Fluidanschlussstutzen
- 12: Ventil
- 14: erster Flanschteil
- 16: zweiter Flanschteil
- 18: Griffteil am äußeren Hülsenteil 6
- 20: Zapfen am äußeren Hülsenteil 6
- 22: geschlitzter Ring
- 24: Einkerbungen in ersten Flanschteil 14
- 26: Gummischeibe
- 28: Körperdecke

## Patentansprüche

1. Trokarhülse für die minimal-invasive Chirurgie, mit einem ersten Hülsenteil (4), der im Wesentlichen die Form eines geraden Rohrstücks mit einer Längsachse hat, und mit einem zweiten Hülsenteil (6), der den ersten Hülsenteil (4) eng anliegend mindestens teilweise umgibt und im Gebrauch relativ zu dem ersten Hülsenteil (4) beweglich ist,
**dadurch gekennzeichnet,**
**dass** die Beweglichkeit des zweiten Hülsenteils (6) relativ zu dem ersten Hülsenteil (4) im Wesentlichen nur in einer Drehbarkeit des zweiten Hülsenteils (6) um den ersten Hülsenteil (4) herum besteht und dass der erste Hülsenteil (4) und der zweite Hülsenteil an ihnen distalen axialen Enden jeweils einen Flanschteil (14, 16) aufweisen, der sich über einen Winkel von weniger als 180° von dem jeweiligen Hülsenteil (4, 6) radial nach außen erstreckt.

2. Trokarhülse nach Anspruch 1, **dadurch gekennzeichnet, dass** der zweite Hülsenteil (6) derart geführt ist, dass er zwischen zwei Endstellungen drehbar ist, die bezüglich der Längsachse um ungefähr 180° auseinander liegen, wobei die eine Endstellung einer Einführposition entspricht, in der die Flanschteile (14, 16) des ersten und des zweiten Hülsenteils (4, 6) in dieselbe Richtung weisen und im Wesentlichen übereinander liegen, und wobei die andere Endstellung einer Arbeitsposition entspricht, in der die Flanschteile (14, 16) des ersten und des zweiten Hülsenteils (4, 6) ungefähr in einander entgegengesetzte Richtungen weisen und ungefähr in einer Ebene senkrecht zu der Längsachse liegen.

3. Trokarhülse nach Anspruch 2, **dadurch gekennzeichnet, dass** die zwei Endstellungen um genau 180° auseinander liegen und/oder dass in der Einführposition die beiden Flanschteile (14, 16) deckungsgleich übereinander liegen und/oder dass in der Arbeitsposition die beiden Flanschteile (14, 16) genau in einer Ebene senkrecht zu der Längsachse liegen.

4. Trokarhülse nach einem der vorhergehenden Ansprüche, **dadurch gekennzeichnet, dass** ein proximales Ende des ersten Hülsenteils (4) an einem Kopfteil (2) befestigt ist, der ggf. eine Dichtung (8) zum gasdichten Einführen eines Instruments in die Trokarhülse und einen Fluidanschlussstutzen (10) aufweist, wobei der Kopfteil (2) Führungs- und Verriegelungsmittel (22) enthält, in denen ein proximales Ende des zweiten Hülsenteils (6) für die Drehung um den ersten Hülsenteil (4) geführt ist und zumindest in der Endstellung des zweiten Hülsenteils (6), die der Arbeitsposition entspricht, verriegelbar ist.

5. Trokarhülse nach einem der vorhergehenden Ansprüche, **dadurch gekennzeichnet, dass** die Flanschteile (14, 16) einstückig mit dem jeweiligen Hülsenteil (4, 6) verbunden sind und/oder jeweils ungefähr 1 mm dick sind.

6. Trokarhülse nach einem der vorhergehenden Ansprüche, **dadurch gekennzeichnet, dass** die Flanschteile (14, 16) ungefähr U-förmige radiale Umrisse haben, wie in einer Ebene senkrecht zu der Längsachse gesehen, wobei der Abstand der beiden Schenkel der U-Form ungefähr gleich dem Durchmesser des ersten (4) oder vorzugsweise des zweiten Hülsenteils (6) ist.

7. Trokarhülse nach einem der vorhergehenden Ansprüche, **dadurch gekennzeichnet, dass** der am ersten Hülsenteil (4) anliegende Abschnitt des zweiten Hülsenteils (6) im Wesentlichen die Form eines geraden Rohrstücks hat.

8. Trokarhülse nach einem der Ansprüche 1 bis 6, **dadurch gekennzeichnet, dass** der am ersten Hülsenteil (4) anliegende Abschnitt des zweiten Hülsenteils (6) im Wesentlichen die Form einer Hälfte eines der Länge nach zweigeteilten Rohres hat.

9. Trokarhülse nach Anspruch 8, **dadurch gekennzeichnet, dass** der Querschnitt des zweiten Hülsenteils (6) senkrecht zu der Längsachse ein Kreisbogen ist, der einige Winkelgrade mehr als ein Halbkreis umfasst.

10. Trokarhülse nach einem der vorhergehenden Ansprüche, **dadurch gekennzeichnet, dass** sie ein scheibenförmiges Konterelement, vorzugsweise in Form einer einstückigen geschlitzten Gummischeibe (26), umfasst, das dafür eingerichtet ist, den ersten und den zweiten Hülsenteil (4, 6) ringförmig zu umschließen und die Flanschteile (14, 16) in Richtung der Längsachse zu kontern.
